# FASCICULE DE BREVET EUROPEEN

(11) **EP 3 101 011 B1**
(45) Date de publication et mention de la délivrance du brevet: **20.06.2018**
(21) Numéro de dépôt: 16176645.6
(22) Date de dépôt: 14.05.2009
(51) Int. Cl.: C07D 227/087, C07D 201/04, C07C 249/06

(54) **PROCÉDÉ DE PRÉPARATION DE LACTAMES COMPRENANT UNE ÉTAPE DE PHOTONITROSATION SUIVIE D'UNE ÉTAPE DE TRANSPOSITION DE BECKMANN**
HERSTELLUNGSVERFAHREN VON LACTAMEN, DAS EINE PHASE DER FOTOCHEMISCHEN NITROSIERUNG GEFOLGT VON EINER PHASE DER BECKMANNSCHEN UMLAGERUNG UMFASST
METHOD FOR PREPARING LACTAMES, COMPRISING A PHOTONITROSATION STEP, FOLLOWED BY A BECKMANN REARRANGEMENT STEP

(30) Priorité: 26.05.2008 FR 0853419
(43) Date de publication de la demande: 07.12.2016
(62) Demande divisionnaire de: 09766032.8
(73) Titulaire: ARKEMA FRANCE, 92700 Colombes (FR)
(72) Inventeur: AUBERT, Thierry, 64230 LESCAR (FR)

(56) Documents cités:
- EP-A- 0 989 118
- WO-A-01/74758
- WO-A-99/01424
- WO-A-2006/136699
- FR-A- 2 417 501
- JP-A- 2001 002 636
- US-A- 3 553 091
- HAJIME MIYAMA ET AL: "Quantum yield of photonitrosation of cyclohexane in homogeneous system", THE JOURNAL OF PHYSICAL CHEMISTRY, vol. 73, no. 12, 1 décembre 1969 (1969-12-01), pages 4345-4347, XP055019198, ISSN: 0022-3654, DOI: 10.1021/j100846a053

## Description

### [Domaine de l'invention]

La présente invention se rapporte à un procédé de préparation de lactames, comprenant notamment une étape de photonitrosation de cycloalcanes en présence de diodes électroluminescentes, LEDs dans la suite du texte (light emitting diodes en anglais) suivie d'une étape de transposition de Beckmann réalisée dans un microréacteur, de préférence dans un microréacteur en verre.

### [L'art antérieur et le problème technique]

On connaît l'utilité industrielle des lactames. A titre d'exemple, le caprolactame et le lauryllactame sont respectivement des précurseurs des polyamides 6 et 12.

Au niveau industriel, un procédé pour synthétiser un lactame à partir d'un cycloalcane peut mettre en oeuvre successivement les deux étapes réactionnelles suivantes :
- dans une première étape réactionnelle, on effectue une photonitrosation du cycloalcane à l'aide de chlorure de nitrosyle (NOCl). Cette photonitrosation est généralement réalisée en milieu biphasique solvant organique/acide sulfurique à l'aide de lampes à mercure ou à sodium. Une oxime est ainsi produite en phase organique et ultérieurement extraite sous forme de chlorhydrate d'oxime par la phase sulfurique ;
- dans une seconde étape réactionnelle, on effectue une transposition de Beckmann du chlorhydrate d'oxime extrait en milieu sulfurique concentré pour obtenir le lactame. Ce lactame résultant de la transposition de Beckmann est ensuite isolé et purifié pour conduire à un produit de haute pureté.
- Ce type de procédé est décrit par exemple dans WO200613699, WO9901424, EP 0989118 et US3553091.

A noter que ce type de procédé n'est pas le plus communément utilisé pour faire des lactames et que généralement, la transposition de Beckmann s'effectue au départ d'une oxime obtenue à partir d'une cycloalcanone et non pas par photonitrosation d'un cycloalcane ;en conséquence, d'une part l'oxime n'est pas sous forme de chlorhydrate mais sous forme « base libre » (non salifiée) ou sulfate, d'autre part, elle ne contient pas d'impuretés chlorées issues de la photonitrosation.

Le procédé décrit ci-dessus comprenant une étape de photonitrosation est plus rapide mais présente des inconvénients. Par exemple, l'étape de photonitrosation est particulièrement coûteuse.

En effet, les lampes à mercure ou à sodium utilisées sont fragiles et elles présentent une faible durée de vie. De plus, elles nécessitent un refroidissement et une alimentation électrique importants, et donc coûteux, compte-tenu de leur très forte puissance.

Par ailleurs, cette étape de photonitrosation est peu sélective et génère de 5 à 10 % de dérivés chlorés, en particulier des chlorhydrates de chloro-oximes, qui sont extraits par l'acide sulfurique conjointement avec le chlorhydrate d'oxime désiré, ainsi que 5 à 10 % d'autres impuretés.

De tels dérivés chlorés sont transformés -réactions dénommées par la suite réactions de déchloration- lors de la seconde étape réactionnelle relative à la transposition de Beckmann, ce qui diminue le rendement de cette transposition.

Ainsi, par déchloration, on entend toute réaction chimique permettant d'éliminer le ou les atomes de chlore liés à un squelette carboné.

En effet, comme les conditions de températures et de temps de séjour nécessaires à la déchloration sont plus sévères que les conditions requises pour effectuer la transposition de Beckmann, il en résulte des réactions secondaires d'hydrolyse du lactame final et du chlorhydrate d'oxime issu de l'étape de photonitrosation.

C'est pourquoi, il est difficile d'obtenir à la fois de bons rendements de transposition et de déchloration dans cette seconde étape réactionnelle. En effet, quels que soient les conditions de température et de temps de séjours étudiés en réacteur traditionnels volumiques, le rendement maximal de transposition du chlorhydrate d'oxime en lactame est de l'ordre de 90% pour un rendement de déchloration de l'ordre de 70%, étant noté que des conditions conduisant àune augmentation du rendement de la déchloration provoquent une baisse, rédhibitoire d'un point de vue économique, du rendement de transposition.

D'autre part, les impuretés chlorées résiduelles, qui n'ont pas été transformées lors de la réaction de déchloration, doivent être éliminées lors d'étapes ultérieures de purification, étapes qui sont d'autant plus coûteuses que la quantité d'impuretés chlorées est importante.

Il convient par ailleurs de noter que la réaction de transposition de Beckmann, qui s'effectue généralement dans l'acide sulfurique concentré à des températures supérieures à 100 °C, est extrêmement exothermique.

Cet environnement est d'autant plus difficile que l'acide chlorhydrique présent dans le milieu, provenant du chlorhydrate d'oxime et/ou dégagé lors de l'élimination des dérivés chlorés, rend le milieu extrêmement corrosif. Cette deuxième étape nécessite donc des matériaux et des dispositifs de sécurité particulièrement coûteux.

Concernant l'utilisation de LEDs pour effectuer des réactions de photochimie, le lecteur trouvera dans les références suivantes quelques états de l'art : « Leuchtdioden in der Chemie- Ein Hochzeit verschiedener Technologien » Chemie Ingenieur Technik (2007), 79(1-2), 153-159 ou « recent progress on photoreactions in microreactors », Pure and Applied Chemistry, 79(11), 1959-1968 (2007), ou encore les travaux de l'équipe du Professeur Teijiro ICHIMURA qui sont centrés sur l'utilisation de LEDs dans le domaine de l'ultraviolet pour réaliser diverses réactions chimiques (réduction, N-alkylation et oxydation principalement) ; voir par exemple : Chemistry Letters,Vol 35 N°4 (2006) p.410 ou « Photoreactions » dans « Microchemical Engineering in Practice », T.R.Dietrich, Ed. Blackwell Publishing (2006).

Concernant plus particulièrement l'utilisation de microréacteurs, le brevet WO0174758 décrit l'utilisation d'un microréacteur pour la transposition de Beckmann de l'oxime (base libre) de l'acétophénone ; dans ce cas, il n'y a pas de réactions de déchloration à effectuer simultanément à la transposition de Beckmann, donc il est aisé d'optimiser le rendement de la réaction.

Dans l'article Studies in Surface Science and Catalysis (2007), 172(Science and Technology in Catalysis 2006), 129-132, est décrite une étude cinétique de la transposition de Beckmann de l'oxime (base libre) en microréacteur ; dans ce cas également, il n'y a pas de réactions de déchloration à effectuer simultanément à la transposition de Beckmann.

La transposition de l'oxime (base libre) du cyclohexane dans un microréacteur métallique est décrite dans l'article Organikum, VEB Deutscher Verlag der Wissenschaften Berlin, 1988, 576 ainsi que dans Chemfiles, vol 5 , n°7 ,publication de Sigma Aldrich; là encore, il n'y a pas de réactions de déchloration à effectuer simultanément à la transposition de Beckmann, donc il est aisé d'optimiser le rendement de la réaction ; par ailleurs, la transposition d'un mélange de chlorhydrates d'oxime et de chloro-oximes qui génère de grandes quantités d'acide chlorhydrique corrosif n'est pas réalisable dans des matériaux métalliques.

Plusieurs articles écrits par le National Institute of Advanced Industrial Science and Technology (Materials Chemistry in Supercritical Fluids (2005), 123-144 , CA : 146:358239 ; International Journal of Chemical Reactor Engineering (2005), 3, No pp. given , CA : 144:88591 ; Gurin Kemisutori Shirizu (2004), 3(Chorinkai Ryutai no Saishin Oyo Gijutsu), 45-67, CA : 142:410655 ; Kurin Tekunoroji (2004), 14(6), 47-50 , CA : 141:123911 ; Petrotech (Tokyo, Japan) (2003), 26(9), 721-725, CA: 140:423523 ; Chemical Communications (Cambridge, United Kingdom) (2002), (19),2208-2209, CA: 138:73570) décrivent l'utilisation de microréacteurs pour la transposition de l'oxime (base libre) de la cyclohexanone en milieu H2O supercritique, donc dans des conditions extrêmes de température et de pression. Cette technologie n'est pas applicable à la transposition du chlorhydrate d'oxime résultant d'une étape de photonitrosation, qui est en solution dans de l'acide sulfurique concentré.

Enfin, la transposition de l'oxime (base libre) de la cyclohexanone dans un microréacteur métallique comportant un catalyseur B2O3/TiO2-ZrO2 en lit fixe est décrit dans Applied Catalysis, A: General (2004), 263(1), 83-89; ce procédé n'est pas non plus applicable à la transposition d'un mélange de chlorhydrates d'oxime et de chloro-oximes car l'acide chlorhydrique généré est incompatible avec le catalyseur utilisé et le matériau du microréacteur.

### [Brève description de l'invention]

La présente invention concerne un procédé de préparation de lactames dans lequel on effectue une photonitrosation d'un cycloalcane à l'aide de chlorure de nitrosyle (NOCI), cette photonitrosation étant effectuée au moyen de LEDs émettant une lumière monochromatique. La combinaison de plusieurs LEDs de différentes longueurs d'ondes n'est pas exclue dans le cadre de l'invention, à condition que chacune des LEDs présente un spectre d'émission monochromatique. En particulier, le procédé de l'invention est défini dans les revendications.

L'utilisation de LEDs présente de nombreux avantages, notamment un coût d'utilisation moindre par rapport aux lampes à mercure ou à sodium. En effet, les LEDs ont une durée de vie supérieure à la durée de vie de ces lampes et requièrent un refroidissement et une consommation énergétique plus faibles.

En outre, les LEDs ne contiennent pas de mercure, produit extrêmement toxique, dont l'utilisation et le recyclage complexe augmentent encore le coût d'utilisation.

Les LEDs présentent également l'avantage d'être une source de lumière monochromatique, avec un spectre d'émission relativement étroit, ce qui permet d'améliorer de manière surprenante la sélectivité de la réaction de photonitrosation.

Par exemple, l'utilisation de LEDs ayant une longueur d'onde de 590 nm permet de gagner de 1 à 2 % de sélectivité pour la réaction de photonitrosation.

Le procédé selon l'invention peut également présenter une ou plusieurs des caractéristiques ci-dessous, considérées individuellement ou selon toutes les combinaisons techniquement possibles :
- selon une réalisation, la lumière monochromatique émise par les LEDs a une valeur de longueur d'onde moyenne comprise dans un intervalle entre 550 et 650 nm ;
- selon une réalisation, la lumière monochromatique émise par les LEDs a une valeur de longueur d'onde moyenne de 585 à 650 nm ;
- selon une autre réalisation, un microréacteur, dont le corps est de préférence en verre, est utilisé pour effectuer la photonitrosation du cycloalcane ;
- selon une autre réalisation, le procédé selon l'invention comprend en outre une étape de transposition de Beckmann du chlorhydrate d'oxime généré lors de cette photonitrosation ;
- selon une variante, on utilise un microréacteur pour effectuer l'étape de transposition de Beckmann, le corps du microréacteur pouvant avantageusement comprendre du tantale, un polymère fluoré, un acier verré ou du verre et étant préférentiellement réalisé en verre. Grâce à cette variante de l'invention, il est possible d'effectuer l'étape de transposition de Beckmann/déchloration dans des conditions de contrôle précis du débit, de température et de temps de séjour des réactifs, ce qui accroît la sécurité et le rendement de cette réaction.
Il est précisé que l'on entend par "corps du microréacteur" la partie du microréacteur qui se trouve au contact du milieu réactionnel, de l'étape de photonitrosation et/ou de l'étape de transposition de Beckmann.

Notamment, il a été constaté que, de façon surprenante, le rendement de la déchloration et le rendement de la transposition de Beckmann étaient améliorés simultanément et ce, indépendamment des conditions de mise en oeuvre de l'étape de photonitrosation préalable du cycloalcane, que celles-ci utilisent des LEDs ou des lampes à mercure ou à sodium de l'art antérieur.

En outre, l'utilisation d'un microréacteur permet d'assurer des conditions de sécurité optimales en raison des faibles volumes de réactifs utilisés et de l'excellent contrôle de l'exothermicité de la réaction.

Selon un aspect plus particulièrement préféré, on utilise un microréacteur réalisé en verre, qui est moins onéreux.

D'autres caractéristiques et avantages de l'invention apparaîtront à la lumière de la description d'une réalisation de l'invention indiquée ci-dessous à titre illustratif et non limitatif.

### [Description détaillée d'une réalisation de l'invention]

Un procédé de préparation de lactames conforme à l'invention comprend l'utilisation de LEDs pour effectuer la photonitrosation d'un cycloalcane, suivi d'une étape de transposition de Beckmann/déchloration réalisée dans un microréacteur, de préférence dans un microréacteur en verre.

On appellera par la suite « microréacteur » tout ensemble de réacteurs microstructurés montés en série ou en parallèle, et « réacteurs microstructurés » ou simplement « microstructure » tout réacteur chimique dont l'une au moins des dimensions caractéristiques est comprise entre 1 micron et 15 millimètres et caractérisé par :
une valeur extrêmement élevée, par rapport aux réacteurs traditionnels volumiques, du rapport entre sa surface et son volume internes, plus précisément de l'ordre de 1000 m²m⁻³ ou plus, contre généralement 100 m²m⁻³ ou moins pour les réacteurs traditionnels.
un volume interne total très faible, généralement compris entre quelques microlitres et quelques dizaines de millilitres,
des faibles temps caractéristiques (temps de séjour, temps de mélange, etc..), généralement inférieurs à environ 10 minutes.

Comme précédemment indiqué, l'utilisation de LEDs permet de réduire le coût du procédé de préparation de lactames compte tenu de leur faible consommation et de leur longue durée de vie comparativement aux lampes à mercure ou à sodium.

En outre, les LEDs ont une taille et une puissance qui permettent leur utilisation dans un microréacteur pour accroître la sélectivité des réactions.

Les LEDs mises en oeuvre dans cette réalisation ont une puissance électrique supérieure à 1 Watt environ et sont caractérisées par une efficacité lumineuse supérieure à 10 lumen/W.

De telles LEDs sont disponibles chez de nombreux fournisseurs tels que Philips Lumileds (gamme Luxeon® par exemple), Cree Inc. ou Nichia Corporation.

Par ailleurs, l'utilisation d'un microréacteur permet une agitation homogène du milieu réactif et assure notamment un meilleur transfert du chlorhydrate d'oxime de la phase organique à la phase sulfurique.

Finalement, le contrôle de la température réactionnelle et du temps de séjour des composés chimiques en présence est plus aisé dans un microréacteur, ce qui est un avantage pour une réaction fortement exothermique telle que la transposition de Beckmann.

Avantageusement, l'une au moins des deux étapes, photonitrosation, d'une part, et transposition de Beckmann/déchloration, d'autre part, est effectuée dans un tel microréacteur.

Dans un mode de réalisation préféré de l'invention, chacune des deux étapes réactionnelles, photonitrosation, d'une part, et transposition de Beckmann/déchloration, d'autre part, est effectuée dans un tel microréacteur.

Concernant la première étape réactionnelle, on effectue la photonitrosation d'un cycloalcane à l'aide de chlorure de nitrosyle (NOCl). Cette photonitrosation est réalisée en milieu biphasique solvant organique/acide sulfurique dans les conditions de températures et de concentrations bien connues de l'homme de l'art telles que celles décrites, par exemple, dans le document EP 0 993 438.

Une oxime est ainsi générée en phase organique, cette oxime étant ensuite extraite sous forme de chlorhydrate d'oxime par la phase sulfurique.

Selon une forme préférée de l'invention, le cycloalcane est le cyclododécane, on obtient alors par photonitrosation le chlorhydrate de cyclododécanone-oxime selon la réaction :

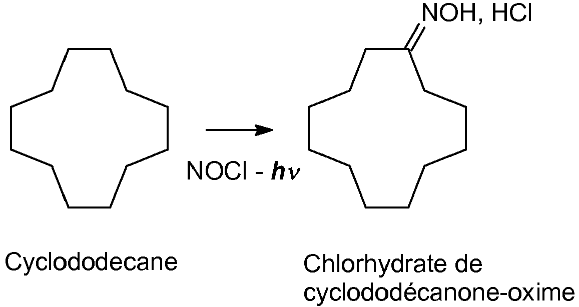

Il convient de noter que, conformément à l'invention, la source de photons (hv) est constituée de LEDs qui émettent de façon monochromatique, c'est-à-dire principalement selon une plage ou un spectre de longueur d'onde significativement plus étroit que la plage d'émission des lampes à mercure ou à sodium, typiquement de l'ordre de 15 à 30 nm.

En conséquence, ce spectre d'émission des LEDs est caractérisé par une longueur d'onde moyenne d'une valeur typiquement comprise dans un intervalle de 550 à 650 nm, dans le cas de la photonitrosation effectuée conformément à l'invention.

Ainsi, une valeur de longueur d'onde moyenne peut être de 585 à 650 nm.

En outre, l'utilisation d'un microréacteur permet une meilleure répartition spatiale des photons émis par les LEDs dans le milieu réactionnel, ce qui est un avantage pour une réaction photochimique telle que la photonitrosation.

Concernant la seconde étape réactionnelle, on effectue une transposition de Beckmann/déchloration du chlorhydrate d'oxime issu de la première étape de photonitrosation, en milieu sulfurique concentré.

Poursuivant la forme préférée de l'invention ci-dessus d'une préparation de lactame à partir de cyclododécane, on obtient le lauryllactame ou dodécalactame selon la réaction :

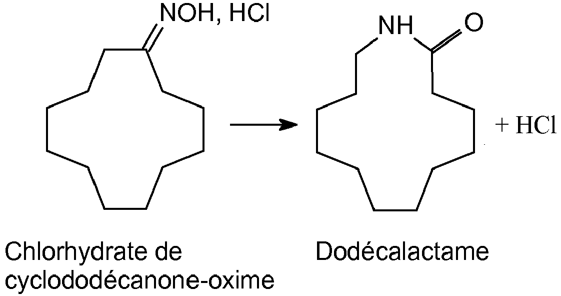

De manière inattendue, l'utilisation de microréacteurs pour l'étape de transposition de Beckmann/déchloration permet une amélioration surprenante à la fois du rendement de déchloration et du rendement de transposition de Beckmann proprement dit grâce au contrôle précis des températures et des temps de séjour, ce qui permet de réduire significativement les réactions secondaires (hydrolyse du chlorhydrate d'oxime et du lactame).

Ainsi, on peut atteindre des rendements de déchloration compris entre 70 et 80 % et des rendements de transposition de Beckmann supérieurs à 90 %, ce qui n'était pas possible en effectuant cette réaction dans des réacteurs volumiques classiques.

Ces résultats inattendus peuvent être obtenus par exemple dans les conditions suivantes :
- soit en une seule étape réactionnelle conduite à des températures comprises entre 160 et 230°C et des temps de séjour compris entre 2 secondes et 10 minutes en fonction du nombre de microréacteurs associés,
- soit en deux étapes réactionnelles : la première, correspondant majoritairement à la transposition de Beckmann, étant conduite à des températures comprises entre 100 et 160°C et des temps de séjour compris entre 2 secondes et 10 minutes en fonction du nombre de réacteurs associés ; la deuxième, correspondant majoritairement aux réactions de déchloration, étant conduite à des températures comprises entre 160 et 240°C et des temps de séjour compris entre 2 secondes et 8 minutes en fonction du nombre de microréacteurs associés.

Par ailleurs, l'utilisation de microréacteurs améliore nettement la sécurité du procédé en raison des faibles volumes mis en jeu et du contrôle particulièrement précis de l'exothermicité.

Les étapes décrites précédemment -transposition de Beckmann et déchloration- sont avantageusement mises en oeuvre dans un microréacteur en verre. En effet, l'utilisation du verre comme matériau permet de résoudre les problèmes de corrosion habituellement observés avec des matériaux classiques tels que des métaux.

Il convient de noter ici que l'utilisation d'un microréacteur requiert des investissements compensés par une amélioration des rendements des différentes réactions de la synthèse de lactames décrites ci-dessus - photonitrosation, et/ou transposition de Beckmann et déchloration.

De même, la diminution des problèmes liés à la corrosion, à la sécurité et aux coûts élevés de ces opérations compense ces investissements.

La technologie permettant de fabriquer ces microréacteurs en verre est aujourd'hui bien connue de l'homme de l'art et on peut utiliser pour effectuer les réactions précédentes un microréacteur en verre fourni, par exemple, par les sociétés Corning ou Invenios.

Typiquement, les microréacteurs utilisés peuvent se présenter sous la forme d'une plaque dont la surface est comprise entre 100 et 2500 cm² ; ils présentent des canaux dont le diamètre est compris entre 50 microns et 10 mm et un système de chauffage par fluide caloporteur permettant d'atteindre des températures pouvant atteindre 250 °C.

Les verres utilisables pour fabriquer ces microréacteurs sont tous les types de verre tels que les verres borosilicates (Pyrex®, par exemple), les verres sodocalciques, les verres au plomb, les verres de silice ou les vitrocéramiques.

### EXEMPLES

### Exemple 1 : Photonitrosation du cyclododécane au moyen de LEDs :

Dans un réacteur de deux litres équipé en son centre d'un faisceau de 80 LEDs LUXEON LXML-PL01-0030 de la société PHILIPS LUMILEDS fournissant chacune 30 lumens (pour un courant de 350 mA) et émettant une lumière monochromatique centrée à 590 nm, on introduit 3806 g d'une solution à 32 % massique de Cyclododécane dans du tétrachlorure de carbone et 200 g d'acide sulfurique à 90 % sous agitation, on allume les lampes puis on introduit en continu 10 l/h d' acide chlorhydrique gazeux anhydre et 10 l/h de chlrorure de nitrosyle pendant 3 h, en refroidissant le milieur réactionnel de telle sorte que la température ne dépasse pas 25°C.

La sélectivité de la réaction, exprimée par le ratio entre le pourcentage d'oxime de la cyclododécanone et la somme du pourcentage d'oxime de la cyclododécanone et des sous-produits de la réaction dosée par HPLC dans la phase sulfurique est de 89 %, supérieure donc à celle observée en utilisant les lampes à vapeur de mercure ou de sodium de l'art antérieur.

### Exemple 2 transposition de Beckmann/déchloration :

- On injecte à un débit de 1 L/h et à température ambiante de l'acide sulfurique 90% avec une pompe d'alimentation Grundfos DME-2-18 dans une microstructure de type « DT » de 9 ml de volume interne (circuit réactif), fabriquée par la société CORNING et décrite par exemple dans l'article : Chem.Eng.Technol. 2008, 31, N°8, 1146-1154 de P.Barthe et Coll.
- Dans le circuit du fluide caloporteur, on injecte à un débit de 6 L/min. de l'huile à 205°C par l'intermédiaire d'un bain Lauda Integral XT 350 HW. Une fois la température de 200 °C atteinte à la sortie de la microstructure dans le circuit du fluide caloporteur, on arrête l'injection d'acide sulfurique et on injecte une solution à 30,1 % (massique) d'oxime de cyclododécanone (déterminé par HPLC) dans de l'acide sulfurique à 90 % à un débit de 2,5 L/h environ grâce à 2 pompes Grundfos DME-2-18 montées en parallèle.
- Après 25 minutes pendant lesquelles ont été injectés 1017,9 g de solution d'oxime, on récupère en sortie du circuit réactif 964,8 g d'une solution de lactame brune dont l'analyse par HPLC est la suivante :
   Lactame : 30,2 % (massique).

Le rendement de transposition est donc de 95,1 %.

Les pourcentages de chlore dans les oximes et lactames solides obtenus par précipitation et lavage des solutions sulfuriques d'oxime et de lactame sont respectivement de 2,18 et 0,65 % ; le rendement de déchloration est donc de 70 %.

### Exemple 3 : transposition de Beckmann/déchloration :

- On injecte à un débit de 1 L/h et à température ambiante de l'acide sulfurique 90% avec une pompe d'alimentation Grundfos DME-2-18 dans 4 microstructures montées en série de type « DT », de 9 ml de volume interne chacune (circuit réactif), fabriquées par la société CORNING et décrite par exemple dans l'article: Chem.Eng.Technol. 2008, 31, N°8, 1146-1154 de P.Barthe et Coll.
- Dans le circuit du fluide caloporteur, on injecte à un débit de 6 L/min. de l'huile à 190°C par l'intermédiaire d'un bain Lauda Integral XT 350 HW. Une fois la température de 185 °C atteinte à la sortie de la microstructure dans le circuit du fluide caloporteur, on arrête l'injection d'acide sulfurique et on injecte une solution à 30,9 % (massique) d'oxime de cyclododécanone (déterminé par HPLC) dans de l'acide sulfurique à 90 % à un débit de 1 L/h environ grâce à 1 pompe Grundfos DME-2-18.
- Après 1 h pendant laquelle ont été injectés 766 g de solution d'oxime, on récupère en sortie du circuit réactif 738 g d'une solution de lactame brune dont l'analyse par HPLC est la suivante :

Lactame : 29,3 % (massique).

Le rendement de transposition est donc de 91,3 %.

Les pourcentages de chlore dans les oximes et lactames solides obtenus par précipitation et lavage des solutions sulfuriques d'oxime et de lactame sont respectivement de 2,62 et 0,48 % ; le rendement de déchloration est donc de 81,7 %.

L'utilisation de microréacteurs montre donc que par rapport à l'art antérieur, on observe une amélioration du rendement de transposition de Beckmann (95,1% contre 90 % dans l'art antérieur) pour un rendement de déchloration de 70%, ou une amélioration de la dechloration (81.7% contre 70% dans l'art antérieur) pour un rendement de transposition de Beckmann de 91.3%.

## Revendications

1. Procédé de préparation de lactames dans lequel on effectue une photonitrosation d'un cycloalcane à l'aide de chlorure de nitrosyle (NOCI), **caractérisé en ce que** cette photonitrosation est effectuée au moyen de LEDs émettant une lumière monochromatique de longueur d'onde moyenne comprise dans un intervalle de 550 à 650 nm.

2. Procédé selon la revendication 1, **caractérisé en ce que** la lumière monochromatique émise par les LEDs a une valeur de longueur d'onde moyenne de 585 à 650 nm.

3. Procédé selon l'une des revendications 1 ou 2, dans laquelle le lactame est le caprolactame ou le lauryllactame.

4. Procédé selon l'une des revendications 1 ou 2, dans laquelle le cycloalcane est le cyclododécane.

5. Procédé selon l'une quelconque des revendications 1 à 4, **caractérisé en ce que** l'on utilise un microréacteur pour effectuer la photonitrosation du cycloalcane.

6. Procédé selon la revendication 5, **caractérisé en ce que** le corps du microréacteur est en verre.

7. Procédé selon l'une quelconque des revendications 1 à 6, **caractérisé en ce qu'**il comprend en outre une étape de transposition de Beckmann/déchloration du chlorhydrate d'oxime généré lors de cette photonitrosation, **caractérisé en ce que** l'on utilise un microréacteur pour effectuer l'étape de transposition de Beckmann/déchloration.

8. Procédé selon la revendication 7, **caractérisé en ce que** le corps du microréacteur pour effectuer l'étape de transposition de Beckmann/déchloration comprend du tantale, un polymère fluoré, un acier verré ou du verre.

9. Procédé selon l'une quelconque des revendications 1 à 8, dans lequel le caprolactame et le lauryllactame sont respectivement des précurseurs des polyamides 6 et 12.

## Patentansprüche

1. Verfahren zur Herstellung von Lactamen, bei dem eine Photonitrosierung eines Cycloalkans mit Nitrosylchlorid (NOCl) durchgeführt wird, **dadurch gekennzeichnet, dass** diese Photonitrosierung mit Hilfe von LEDs, die ein monochromatisches Licht mit einer mittleren Wellenlänge in einem Intervall von 550 bis 650 nm emittieren, durchgeführt wird.

2. Verfahren nach Anspruch 1, **dadurch gekennzeichnet, dass** das von den LEDs emittierte monochromatische Licht eine mittlere Wellenlänge von 585 bis 650 nm aufweist.

3. Verfahren nach Anspruch 1 oder 2, bei dem es sich bei dem Lactam um Caprolactam oder Lacryllactam handelt.

4. Verfahren nach Anspruch 1 oder 2, bei dem es sich bei dem Cycloalkan um Cyclododecan handelt.

5. Verfahren nach einem der Ansprüche 1 bis 4, **dadurch gekennzeichnet, dass** man zur Durchführung der Photonitrosierung des Cycloalkans einen Mikroreaktor verwendet.

6. Verfahren nach Anspruch 5, **dadurch gekennzeichnet, dass** der Körper des Mikroreaktors aus Glas ist.

7. Verfahren nach einem der Ansprüche 1 bis 6, **dadurch gekennzeichnet, dass** es außerdem einen Schritt der Beckmann-Umlagerung/Dechlorierung des bei dieser Photonitrosierung gebildeten Oximhydrochlorids umfasst, **dadurch gekennzeichnet, dass** man zur Durchführung des Schritts der Beckmann-Umlagerung/Dechlorierung einen Mikroreaktor verwendet.

8. Verfahren nach Anspruch 7, **dadurch gekennzeichnet, dass** der Körper des Mikroreaktors zur Durchführung des Schritts der Beckmann-Umlagerung/Dechlorierung Tantal, ein Fluorpolymer, mit Glas ausgekleideten Stahl oder Glas umfasst.

9. Verfahren nach einem der Ansprüche 1 bis 8, bei dem Caprolactam und Lauryllactam Vorstufen von Polyamiden 6 bzw. 12 sind.

## Claims

1. Process for preparing lactams, in which a photonitrosation of a cycloalkane is carried out using nitrosyl chloride (NOCl), **characterized in that** this photonitrosation is carried out by means of LEDs emitting a monochromatic light having an average wavelength included in a range of from 550 to 650 nm.

2. Process according to Claim 1, **characterized in that** the monochromatic light emitted by the LEDs has an average wavelength value of from 585 to 650 nm.

3. Process according to either of Claims 1 and 2, in which the lactam is caprolactam or lauryllactam.

4. Process according to either of Claims 1 and 2, in which the cycloalkane is cyclododecane.

5. Process according to any one of Claims 1 to 4, **characterized in that** a microreactor is used to carry out the photonitrosation of the cycloalkane.

6. Process according to Claim 5, **characterized in that** the body of the microreactor is made of glass.

7. Process according to any one of Claims 1 to 6, **characterized in that** it also comprises a step of Beckmann rearrangement/dechlorination of the oxime hydrochloride generated during this photonitrosation, **characterized in that** a microreactor is used to carry out the Beckmann rearrangement/dechlorination step.

8. Process according to Claim 7, **characterized in that** the body of the microreactor for carrying out the Beckmann rearrangement/dechlorination step comprises tantalum, a fluoropolymer, a glass steel or glass.

9. Process according to any one of Claims 1 to 8, in which caprolactam and lauryllactam are precursors of polyamides 6 and 12, respectively.
